# EUROPÄISCHE PATENTSCHRIFT

(11) **EP 0 927 760 B1**
(45) Veröffentlichungstag und Bekanntmachung des Hinweises auf die Patenterteilung: **30.12.2009**
(21) Anmeldenummer: 98122351.4
(22) Anmeldetag: 25.11.1998
(51) Int. Cl.: C12N 15/38, C07K 14/04, C12Q 1/68, G01N 33/53

(54) **Immunreaktive Bereiche des Glycoproteins gpII des Varicella Zoster-Virus (VZV)**
Immunreactive parts of the glycoproteins gpII from Varicell-Zoster-Virus
Parties immunoréactives des glycoprotéines gpII du virus Varicalla-Zoster

(30) Priorität: 23.12.1997 DE 19757767
(43) Veröffentlichungstag der Anmeldung: 07.07.1999
(73) Patentinhaber: Siemens Healthcare Diagnostics Products GmbH, 35041 Marburg (DE)
(72) Erfinder: Eickmann, Markus Dr., 35041 Michelbach (DE); Glicklhorn, Dorothee, 35075 Gladenbach (DE); Radsak, Klaus Prof. Dr., 35037 Marburg (DE); Hauser, Hans-Peter Dr., 35041 Elnhausen (DE); Giesendorf, Bernhard Dr., 35041 Michelbach (DE)

(56) Entgegenhaltungen:
- EP-A- 0 210 931
- EP-A- 0 353 809
- KJARTANSDOTTIR A ET AL: "B-cell epitopes of varicella-zoster virus glycoprotein II." ARCHIVES OF VIROLOGY, Bd. 141, Nr. 12, 1996, Seiten 2465-2469, XP000916937 ISSN: 0304-8608
- KOROPCHAK C M ET AL: "INVESTIGATION OF VARICELLA-ZOSTER VIRUS INFECTION BY POLYMERASE CHAIN REACTION IN THE IMMUNOCOMPETENT HOST WITH ACUTE VARICELLA" JOURNAL OF INFECTIOUS DISEASES, Bd. 163, Nr. 5, 1991, Seiten 1016-1022, XP000916942 ISSN: 0022-1899
- DATABASE WPI Section Ch, Week 199511 Derwent Publications Ltd., London, GB; Class B04, AN 1995-077153 XP002143444 & JP 07 000199 A (TEIJIN LTD), 6. Januar 1995 (1995-01-06)
- HAYWARD A R: "T-CELL RESPONSES TO PREDICTED AMPHIPATHIC PEPTIDES OF VARICELLA-ZOSTER VIRUS GLYCOPROTEINS II AND IV" JOURNAL OF VIROLOGY, Bd. 64, Nr. 2, 1990, Seiten 651-655, XP000917048 ISSN: 0022-538X

## Beschreibung

Die vorliegende Erfindung betrifft immunchemische Verfahren zum Nachweis von Antikörpern gegen Varicella-Zoster-Virus (VZV) unter Verwendung immunreaktiver Peptide, welche dem Bereich der Aminosäurepositionen 450 bis 655 des Glykoproteins II des Varicella-Zoster-Virus entsprechen. Bevorzugt sind dabei solche Verfahren, bei denen besagter Bereich den Aminosäuren 505 bis 647, 517 bis 597 oder 535 bis 584 entspricht. Die vorliegende Erfindung betrifft außerdem Testbestecke enthaltend immunreaktive Peptide entsprechend den vorgenannten Bereichen.

Das VZV wird gemäß der Klassifikation des Internationalen Komitees für Virustaxonomie (ITCV) der Familie der Herpesviridae zugeordnet. Die Primärinfektion erfolgt in 75% der Fälle bis zum 15. Lebensjahr und verläuft meist inapparent. Im Gegensatz dazu kann die Infektion von Erwachsenen, die noch keinen Kontakt mit dem Virus hatten, und bei natürlich oder therapeutisch immunsupprimierten Personen mit schwerer Symptomatik verlaufen. Zu ebenfalls schwerer Symptomatik führt die Infektion des Neugeborenen, da das Virus placentagängig ist und zu diesem Zeitpunkt keine Protektion durch matemale Antikörper besteht. Im Anschluß an die Primärinfektion persistiert das Virus lebenslang in sensorischen Ganglien. Nach Reaktivierung breitet sich das VZV über die peripheren Nerven in sensorischen Ganglien aus und führt dann zum Herpes-Zoster.

Aus der vollständig bestimmten Nukleotidsequenz des VZV-Genoms mit einer Länge von 124.884 bp (Stamm Dumas; (A. J. Davison & J. E. Scott. (1986), J. Gen. Virol. 67, 1759-1816)) sind 70 offene Leserahmen (ORF) deduzierbar, darunter auch die offenen Leserahmen der für die derzeit bekannten Glykoproteine gpI (ORF 68), gpII (ORF 31), gpIII (ORF 37), gpIV (ORF 67), gpV(ORF 14) und gpVI (ORF 60). Die aus der Nukleotidsequenz abgeleitete Aminosäuresequenz zeigt jeweils unterschiedlich ausgeprägte Homologien zu den Glykoproteinen gE, gB, gH, gI, gC, und gL des Herpes Simplex Virus (HSV). Es gibt aber keine Anhaltspunkte dafür, daß aus der Sequenzhomologie auch eine Funktionshomologie abgeleitet werden kann. Die offenen Leserahmen der Glykoproteine gpI, gpII, gpIII und gpV konnten molekularbiologisch bestätigt werden. Im Gegensatz zu dem ausführlicher untersuchten Glykoprotein gB des HSV liegen über das homologe Protein von VZV, gpII, weniger Daten vor. Die entsprechenden Untersuchungen beschränkten sich auf die Biosynthese des gpII und die Bedeutung des gpII für den Vorgang der Virusneutralisation (P. M. Keller et al. (1986), Virologie 152, 181-191; M. Massaer et al. (1993), J. Gen. Virol. 74, 491-494).

Ellis et al. (E.P. 0210931B1) bestätigte den ORF 31 des gpII. Ferner beschreiben Ellis et al. die Reinigung von gpII aus VZV-infizierten MRC-5-Zellen (humane Fibroblasten) und dessen Verwendung zur Herstellung von virusneutralisierenden Antikörpern aus Meerschweinchen. P. M. Keller et al. (1986), Virologie 152, 181-191 und M. Massaer et al. (1993), J. Gen. Virol. 74, 491-494 verwenden unter anderem gpII, das mittels Affinitätschromatographie aus VZV-infizierten Zellen gereinigt wurde, zur Bestimmung des VZV-spezifischen Antikörpertiters humaner Seren. A. Bollen (E.P 0405867 A1; US 371772) exprimiert, nach Deletion des carboxyterminalen Bereichs, membranankerloses gpII mit Hilfe des Baculovirus-Expressionssystems in SF9-Zellen und mittels konstitutiver Expression in CHO-Zellen zur Herstellung einer VZV-Vaccine. Ferner zeigen E. H. Wasmuth & W. J: Miller ((1990), J. Med. Virol. 32, 189-193) unter Verwendung von aus VZV-infizierten Zellen affinitätsgereinigten Glykoproteinen (auch gpII) im Glykoprotein- oder Dot-ELISA die Sensitivität dieser Testmethoden und die Korrelation der ermittelten Antikörpertiter mit der Protektion vor einer VZV-Infektion. Jedoch erlaubt keine der oben erwähnten Publikationen einen Aufschluß über die Herstellung ausreichender Mengen des Glykaproteins gpII zur Etablierung eines Testes im diagnostisch relevanten Maßstab.

Eine Untersuchung fraglich immunreaktiver Bereiche des Glycoproteins gpII wurde von Kjartansdottir et al. (1996), Arch. Virol., Bd. 141, Nr. 12, 2465-2469 vorgestellt. Als Ergebnis dieser Untersuchung konnte von den Autoren der Publikation, abgesehen von einem einzigen Bereich (AS 417-447), kein weiterer immunreaktiver Bereich spezifiziert werden.

Die serologischen Methoden zur Überprüfung der VZV-spezifischen Immunitätslage sind sehr vielfältig und reichen vom Radio- immuno- assay (RIA), Enzyme- linked- immunosorbentassay (ELISA), Fluoreszent- antibody- membran- antigen- assay (FAMA), Immun-Fluoreszenz- Test (IFT) bis zur Komplement- Fixation (CF). Es werden dabei überwiegend VZV spezifische Antikörper nachgewiesen. Als Antigen wird häufig Virusmaterial eingesetzt, welches nach aufwendiger Kultivierung auf humanen Fibroblastenkulturen gewonnen und nach speziellen Methoden für den diagnostischen Einsatz aufbereitet wurde.

Die Gewinnung von VZV-Antigenen aus infizierten Fibroblasten ist für das damit beschäftigte Personal aber mit einer Infektionsgefahr verbunden. Ferner ist die Herstellung sehr kosten- und zeitaufwendig, unter anderem deswegen, weil das Virus von den infizierten Zellen nicht freigesetzt wird und daher spezielle Reinigungsverfahren erforderlich werden. Für die Verwendung des Antigens ohne vorherige Reinigung für einen immunchemischen Test werden die VZV-infizierten Zellen durch Ultraschallbehandlung aufgeschlossen und das Antigen direkt nach Verdünnung für die Beschichtung von beispielsweise Mikrotitrationsplatten eingesetzt. Da bei dieser Methode neben virusspezifischen Antigenen überwiegend auch zellspezifische Antigene an die Mikrotitrationsplattenkavitäten gebunden werden, können die zellspezifischen Antigene, besonders beim Vorliegen bestimmter Krankheiten, beispielsweise Autoimmunkrankheiten, zu falsch-positiven Ergebnissen und damit zu Fehldiagnosen führen. Andere Testverfahren, die auf gereinigten viralen Glykoproteinen basieren, wie beispielsweise ein Glykoprotein-ELISA, bedürfen ausgesprochen aufwendiger und verlustreicher Reinigungsverfahren, so daß die Etablierung immunchemischer diagnostischer Tests im größeren Maßstab bisher kaum durchführbar ist. Aufgrund der ausgeprägten Homologie zwischen den Glykoproteinen der α-Herpesviren sind auch für den Glykoprotein-ELISA Kreuzreaktivitäten mit HSV-spezifischen Antikörpern und dadurch bedingt falsch-positive Resultate häufig zu beobachten.

Eine mögliche Lösung der oben aufgeführten Probleme liegt in der die Verwendung rekombinanter Proteine, die im heterologen System, z. B. in *Escherischia coli (E. coli),* in großer Menge hergestellt werden können. In diesem System ist eine mögliche VZV-Infektion des Personals ausgeschlossen. Ferner ist die Möglichkeit einer auf spezifische Virusproteine ausgerichteten Differentialdiagnostik gegeben. Insbesondere kann der reaktive Bereich der VZV-Proteine soweit eingegrenzt werden, daß Kreuzreaktivitäten mit für andere Herpesviren spezifischen Antikörpern nahezu oder völlig ausgeschlossen werden können. Die diese Voraussetzungen erfüllenden Proteine können auch als Hybridproteine exprimiert werden, wobei entweder ein wirtseigenes Protein, beispielsweise das Maltosebindungsprotein (MBP) von *E. coli* oder eine N-terminal gelegene Sequenz aus 6 Histidinresten (His-tag) als Fusionspartner zur Stabilisierung des Expressionsprodukts beitragen. Diese Hybridproteine können dann über Einstufen-Affinitätsreinigungen in annähernd reiner und damit kontaminationsfreier Form direkt für die Beschichtung von diagnostisch verwendbaren Oberflächen, beispielsweise ELISA-Mikrotitrationsplatten, verwendet werden. Proteine, die die oben genannten Kriterien erfüllen, sind für das VZV-System bisher noch nicht beschrieben worden.

Überraschenderweise konnten auf dem Glycoprotein gpII des VZV diagnostisch verwendbare Epitope lokalisiert werden, die im Bereich der Aminosäurepositionen 450 bis 655 liegen.

Die Gegenstände vorliegender Erfindung werden in den Patentansprüchen definiert. Unter anderem ist dementsprechend Gegenstand der vorliegenden Erfindung ein immunchemisches Verfahren, welches den Nachweis von Antikörpern gegen VZV ermöglicht. In diesem Verfahren wird ein immunreaktives Peptid, ausgewählt aus den Bereichen AS 450 bis 655, AS 505 bis 647, AS 517 bis 597 und AS 535 bis 584, mit einer Probe, beispielsweise einer Blut-, Plasma- oder Serumprobe eines Patienten, in Kontakt gebracht, welche auf das Vorhandensein VZV-spezifischer Antikörper untersucht werden soll. Es wird daraufhin mit dem Fachmann geläufigen Methoden festgestellt, ob Antikörper aus der Probe an die eingesetzten immunreaktiven Peptide binden oder mit diesen in eine andere immunologische Wechselbeziehung treten, beispielsweise eine Kompetition.

### Detaillierte Beschreibung der Erfindung:

Zur Lokalisation diagnostisch verwendbarer immunreaktiver Bereiche wurde die Expression des Glykoproteins II (gpII) in fragmentierter Form im bakteriellem System angewandt. Es ist bekannt, daß nach VZV-Infektion zuerst die gpII-spezifische Immunantwort auftritt. Erst danach erfolgt die Immunantwort gegen gpI und gpIII. Allerdings lagen bisher weder Erkenntnisse über die bakterielle Expression, noch über immunrelevante Epitope des gpII vor. Der gesamte von Ellis et al. (E.P. 0210931B1) beschriebene ORF31 des gpII wurde aus genomischer VZV-DNS (Stamm Ellen) amplifiziert und in den prokaryotischen Expressionsvektor pMAL-p2 (NEB), der die Expression des inserierten DNS-Fragmentes als MBP-Hybridprotein erlaubt, kloniert. Der vollständige ORF31 eignete sich jedoch nicht zur bakteriellen Expression. Aus diesem Grund wurde die Expression von Teilbereichen des ORF31 im pMAL-System untersucht. Bei allen Fragmenten (Fragmentgröße zwischen 400 und 600 bp) konnten entsprechende Expressionsprodukte nachgewiesen werden. Die Reaktivität der Expressionsprodukte wurde mit Hilfe eines aus 25 VZV-reaktiven Humanseren bestehenden Pool-Serum überprüft. Nur eines der Fragmente erwies sich als immunreaktiv. Dabei handelt es sich um ein 206 AS großes Fragment (Seq.1), das zu den AS 450-655 des gpII homolog ist. Zur weiteren Eingrenzung des immunreaktiven Epitops wurden Teilbereiche dieses Fragments zur Expression im pMAL-System verwendet. Anhand des Immunoscreenings konnte gezeigt werden, daß die Fragmente, die die AS 505- 647 (pMAL-427-gpII), AS 517- 597 (pMAL-240-gpII) oder AS 535- 584 (pMAL-149-gpII) kodieren immunreaktiv sind. Der Bereich von AS 535-584 konnte als immunreaktives Hauptepitop des gpII eingestuft werden.

**Sequenz 1:** Aminosäurerest 450 - 655 von gpII (Stamm Ellen). Insgesamt 206 AS; Theoretisches Molekulargewicht 23,5 kDa. Der hervorgehobene Bereich entspricht der immunreaktiven Domäne. Die unterstrichen AS ist die im Vergleich zum Stamm Dumas substituierte AS. Die Numerierung der Aminosäuren beginnt beim Methionin (Startkodon) der publizierten Sequenz des gpII (A. J. Davison & J. E. Scott. (1986), J. Gen. Virol. 67, 1759-1816).

Die entsprechende Nukleotidsequenz ist in Tab. 1 dargestellt.

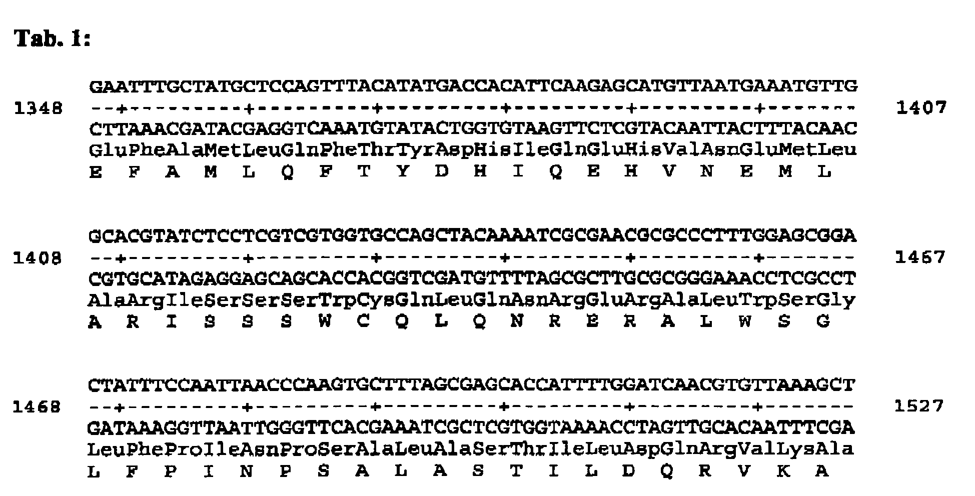

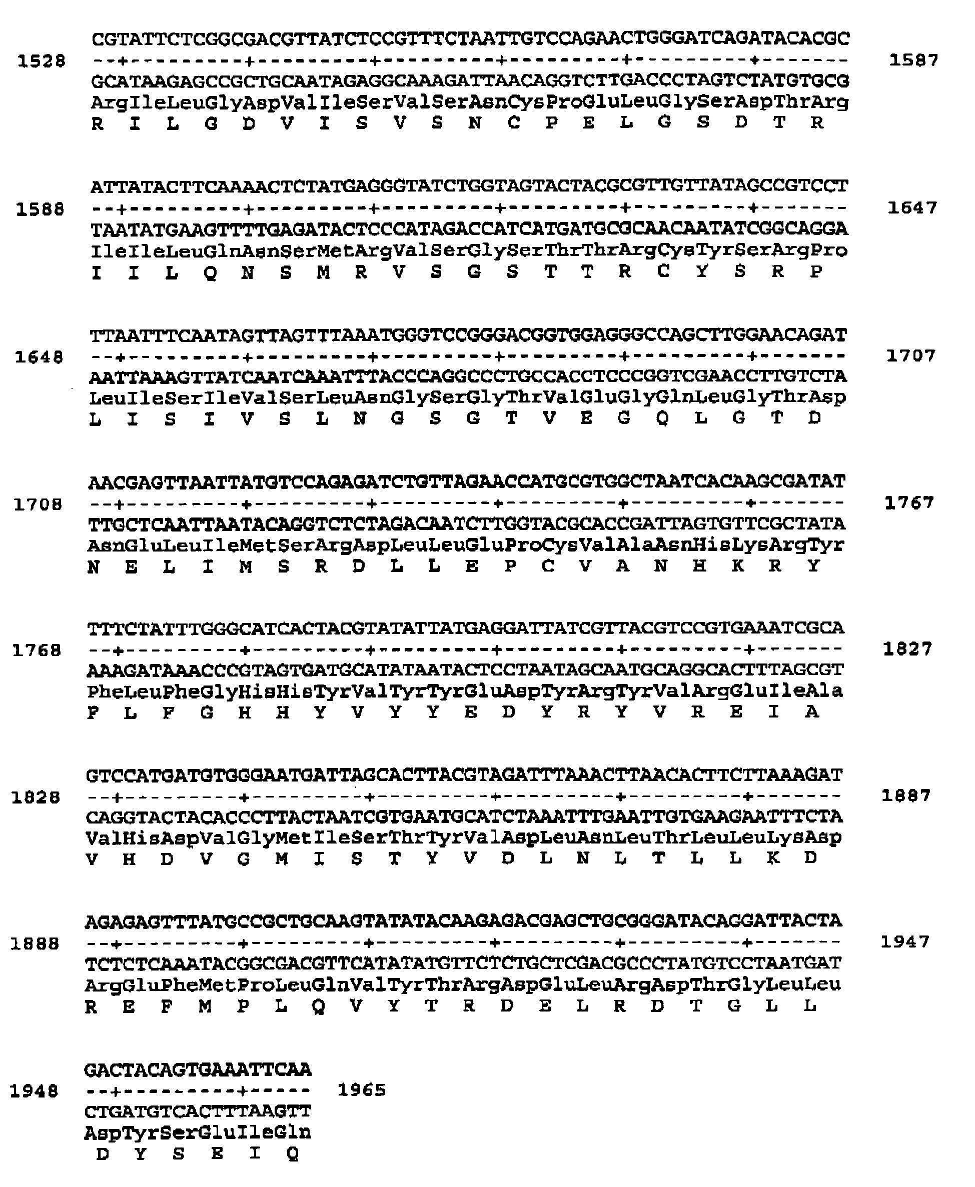

Zur Bewertung dieser Domäne in immunologischen Testverfahren wurde die gpII-Sequenz, die den 206 AS langen Bereich kodiert, in den Vektor pQE (Qiagen) subkloniert. Das Vektorkonstrukt wurde als pQE-gpII* bezeichnet. Dieser Vektor verfügt über einen C-terminal gelegenen Histidin-tag (6 x His) als Fusionsanteil, so daß die Immunreaktivität der zu testenden humanen Seren alleine auf den gpII-Anteil zurückzuführen ist und falsch-positive Resultate in der Immunotestung von VZV-reaktiven Seren ausgeschlossen werden können.

Bei einer Bewertung im ELISA konnte mittels des gereinigten Proteins, welches von dem Plasmid pQE-gpII* kodiert wird, mit dem errechneten cut off von 305 (Mittelwert der Negativseren + 3mal die Standardabweichung) eine Sensitivität von 71% (n=49) und eine Spezifität von 100% (n=5) bzw. mit dem frei gewählten cut off von 160 eine Sensitivität von 82% und eine Spezifität von 80% für die IgG-Diagnostik ermittelt werden.

Bei einer alternativen Bewertung im ELISA konnte mittels des gereinigten Proteins, welches von dem Plasmid pQE-gpII* kodiert wird, mit einem errechneten cut off von 102 (Mittelwert der Negativseren + 3mal die Standardabweichung) eine Sensitivität von 23% (n=13) und eine Spezifität von 100% (n=41) bzw. mit einem frei gewählten cut off von 70 eine Sensitivität von 61,5% und eine Spezifität von 80% für die IgM-Diagnostik ermittelt werden. Sensitivität und Spezifität können jederzeit nach dem Fachmann bekannten Verfahren optimiert werden.

Die vorliegende Erfindung wird nachfolgend außerdem durch Beispiele illustriert, die jedoch in keiner Weise als Einschränkung betrachtet werden sollen.

### Beispiele

### Beispiel 1:

### Gewinnung viraler VZV-Virionen und DNS-Extraktion:

Aus VZV-infizierten Fibroblasten wurden Virionen mittels Ultraschallbehandlung freigesetzt. Die Viren wurden über einen linearen Sucrose-Gradienten (20-70% (w/v)) von zellulären Bestandteilen gereinigt. Nach DNase I Inkubation wurde die virale DNS mittels Proteinase K und Sodiumdodecylsulfat (SDS) Behandlung aus den Virionen freigesetzt. Nach Phenol/Chloroform-Extraktion erfolgte die Fällung der viralen DNS mittels Ethanol.

### Beispiel 2:

### Klonieren des ORF31 und Subklonierung des immunreaktiven Fragments:

Die virale DNS (siehe Beispiel 1) diente als Matritze für die Amplifikation des ORF31, der das gpII kodiert. Als Amplifikationsoligonukleotide dienten folgende Primer:
gpIIH 5' GGAATTCCTTCTATGTTTGTTACGGCGGTTG 3';
gpIIR 5' GCTCTAGAGCATTTACACCCCCGTTACATTCTCG 3'.

Diese Oligonukleotide sind dem entsprechenden Sequenzabschnitt der publizierten Sequenz (A. J. Davison & J. E. Scott. (1986), J. Gen. Virol. 67, 1759-1816) komplemetär, enthalten aber an ihren 5' Termini eine Restriktionsschnitt-stellensequenz, die nicht mit der Matritzen-DNS hybridisierte. Nach erfolgter Amplifikation wurde das 2630 bp große Amplifikat mit den Restriktionsenzymen EcoRI und XbaI terminal geschnitten und in den zuvor mit EcoRI und XbaI linearisierten Expressionsvektor pMAL-p2 ligiert (pMAL-p2-ORF31). Der gesamte ORF31 wurde in überlappender bidirektionaler Weise sequenziert. Er wies eine Basen-Substitutionen an Nukleotidposition 1550 von Cytosin nach Thymidin auf, die zu einer Substitution von Serin zu Phenylalanin auf Aminosäureebene führte.

Zur Subklonierung des immunreaktiven Breiches wurde der inserierte Bereich mit den oben genannten Restriktionsenzymen aus dem Vektor pMAL-p2-ORF31 herausgeschnitten, gelgereinigt und anschließend mit ApoI verdaut. Das resultierende 610 bp große ApoI-Fragment wurde ebenfalls gelgereinigt und in die EcoRI-Schnittstelle des Vektors pMAL-c2 subkloniert. Der Vektor wurde mit pMAL-gpII* bezeichnet. Nach Bestätigung der Immunreaktivität wurde der kodierende Bereich in den Vektor pQE subkloniert. Dazu wurde das 610 bp große Fragment durch die Restriktionsenzyme XmnI und HindIII aus dem Vektor pMAL-gpII* herausgeschnitten und in die SmaI/ HindIII Restriktionsschnittstellen des Vektors pQE32 subkloniert. Der Vektor wurde als pQE-gpII* bezeichnet.

### Beispiel 3:

### Subklonierung des immunreaktiven Epitops:

Durch Amplifikation mit dem Vektor pMAL-gpII* als Matritzen-DNS und den Oligonukleotiden:
5'CGGGATCCCGTGTTAAAGCTCGTATTCTCGGCGACG3' und
5'CCCAAGCTTTAATCCTGTATCCCGCAGCTCGTCTCT3',
5 'CGGGATCCGTTTCTAATTGTCCAGAACTGGGATCAG3' und
5'CCCAAGCTTATACGTAGTGATGCCCAAATAGAAAA3' oder
5'CGGGATCCTCTATGAGGGTATCTGGTAGTACTACGCGTT3' und
5'CCCAAGCTTAGCCACGCATGGTTCTAACAGATC3'
   konnte ein 427 bp, ein 240 bp oder ein 149 bp großes Fragment erhalten werden, das jeweils nach Restriktionsverdauung mit den Enzymen BamHI und HindIII in den mit BamHI und HindIII linearisierten Vektor pMAL-c2 subkloniert wurde. Die resultierenden Konstrukte wurde als pMAL-427-gpII, pMAL-240-gpII oderpMAL-149-gpII bezeichnet.

### Beispiel 4:

### a) Herstellung des rekombinanten Proteins pQE gpII*:

Die Expression und Reinigung des rekombinanten Proteins pQE gpII erfolgte durch Metal Affinity Chromatography unter denaturierenden Bedingungen nach Angaben des Herstellers (Fa. Clontech, Talon Metal Affinity Resin, PT1320-1).

### b) Herstellung der Festphase I (Erfindungsgemäßes System):

Mikrotitrationsplatten Typ B (Fa. Greiner) wurden mit 110 µl je Vertiefung Beschichtungslösung (4 µg/ml rekombinantes pQE gpII in 50 mM Natriumcarbonat-Puffer, pH 9.5) für 18 Stunden bei 4°C inkubiert. Die Vertiefungen der Mikrotitrationsplatten wurden dann dreimal mit je 300 µl Waschlösung (50mMTris/EDTA pH 7,0-7,4 + 0,5%BSA) gewaschen.

### c) Enzymimmunoassay zum Nachweis von VZV-IgG Antikörpern:

Für den Nachweis von Anti VZV IgG im Enzymimmunoassay wurden je 100 µl der 1:100 vorverdünnten Seren in POD Probenpuffer (Behring Diagnostics GmbH, OWBE 945) in der nach Beispiel 4b hergestellten Mikrotitrationsplatte einpipettiert und für 1h bei 37°C inkubiert.
Nach 3maligem Waschen mit Waschlösung POD (Behring Diagnostics GmbH, OSEW 965) wurden 100 µl des Konjugates Anti-Human IgG/POD (Behring Diagnostics GmbH Ch. 243713), 1:50 verdünnt in Microbiol-Konjugatepuffer (Behring Diagnostics GmbH, OUWW 935), einpipettiert. Mit drei weiteren Waschschritten wurde die 1 stündige Inkubation (37°C) beendet. Die gebundene Peroxidase-Aktivität, die direkt mit der Anzahl der gebundenen VZV-Antikörper korreliert, wurde durch Zugabe von H₂O₂/ Tetramethylbenzidin (Behring Diagnostics GmbH OUVG 945) bestimmt. Der Substratumsatz wurde nach 30 Minuten bei Raumtemperatur durch Zugabe von 0.5 M Schwefelsäure (Behring Diagnostics GmbH, OSFA 965) gestoppt und die Extinktion bei 450 nm gemessen.

Anti-VZV-antikörperpositive sowie anti-VZV-antikörpernegative Seren wurden sowohl im Referenzsystem Enzygnost Anti VZV/IgG (Behring Diagnostics GmbH, OWLT 155), wie auch im erfmdungsgemäßen Enzymimmunoassay untersucht. Die Resultate (Extinktionseinheiten) der Untersuchung finden sich in der Tabelle 2. Bei einer Bewertung im ELISA konnte mittels des gereinigten Proteins pQE-gpII* mit dem errechneten cut off von 331 (Mittelwert der Negativseren + 3mal die Standardabweichung) eine Sensitivität von 69% (n=52) und eine Spezifität von 94% (n=16) bzw. mit dem frei gewählten cut off von 335 eine Sensitivität von 69% (n=52) und eine Spezifität von 100% (n=16) für die IgG-Diagnostik ermittelt werden.

### Beispiel 5:

### Enzymimmunoassay zum Nachweis von VZV-IgM Antikörpern:

Der Enzymimmunoassay zum Nachweis von Anti-VZV IgM wurde wie folgt durchgeführt:
Die Seren wurden zuvor 1:50 in POD Probenpuffer (Behring Diagnostics GmbH, OWBE 945) verdünnt und anschließend für 15 Minuten 1:2 mit RF-Adsorbens (Behring Diagnostics GmbH, OUCG 945) bei Raumtemperatur vorinkubiert. In die Vertiefungen der nach Beispiel 4b hergestellten Mikrotitrationsplatten wurden je 100 µl der wie oben beschriebenen vorverdünnten Seren für 1h bei 37°C inkubiert.

Nach dreimaligem Waschen mit Waschlösung POD (Behring Diagnostics GmbH, OSEW 965) wurden 100 µl des Konjugates Anti-Human IgM/POD (Behring Diagnostics GmbH Ch. 4241313), 1:25 verdünnt in Microbiol-Konjugatepuffer (Behring Diagnostics GmbH, OUWW 935), einpipettiert. Mit drei weiteren Waschschritten wurde die 1 stündige Inkubation (+37°C) beendet. Die gebundene Peroxidase-Aktivität, die direkt mit der Anzahl der gebundenen VZV-Antikörper korreliert, wurde durch Zugabe von H₂O₂/ Tetramethylbenzidin (Behring Diagnostics GmbH OUVG 945) bestimmt. Der Substratumsatz wurde nach 30 Minuten bei Raumtemperatur durch Zugabe von 0.5 M Schwefelsäure (Behring Diagnostics GmbH, OSFA 965) gestoppt und die Extinktion bei 450 nm gemessen..

Anti-VZV positive sowie anti-VZV negative Seren wurden sowohl im Referenzsystem Enzygnost Anti VZV/IgM (Behring Diagnostics GmbH, OWLW 155), wie auch im erfindungsgemäßen Enzymimmunoassay untersucht. Die Resultate (Extinktionseinheiten) der Untersuchung finden sich in der Tabelle 3. Bei einer Bewertung im ELISA konnte mittels des gereinigten Proteins pQE-gpII* mit einem errechneten cut off von 102 (Mittelwert der Negativseren + 3mal die Standardabweichung) eine Sensitivität von 38% (n=13) und eine Spezifität von 100 % (n=41) bzw. mit einem frei gewählten cut off von 96 eine Sensitivität von 38% (n=13) und eine Spezifität von 100% (n=41) für die IgM-Diagnostik ermittelt werden.

**Tabelle 2:**

| **Nr.** | **Status** | **ELISA IgG Referenz** | **Elisa IgG pQE gpll 4 µg/ml** |
|---|---|---|---|
| | | **Konj. 1:50** | **Konj. 1:50** |
| | | **Serum 1:231** | **Serum 1:100** |
| 1 | + | 1014 | 963 |
| 2 | + | 302 | 174 |
| 3 | + | 642 | 746 |
| 4 | + | 612 | 674 |
| 5 | + | 1383 | 419 |
| 6 | + | 930 | 1722 |
| 7 | + | 653 | 729 |
| 8 | + | 915 | 1086 |
| 9 | + | 570 | 607 |
| 10 | + | 1770 | >2500 |
| 11 | + | 990 | 222 |
| 12 | + | 698 | 427 |
| 13 | + | 141 | 249 |
| 14 | + | 338 | 590 |
| 15 | + | 556 | 1074 |
| 16 | + | 791 | 615 |
| 17 | + | 2982 | >2500 |
| 18 | + | 1205 | 1736 |
| 19 | + | 694 | 1157 |
| 20 | + | 1143 | 1134 |
| 21 | + | 1405 | 1509 |
| 22 | + | 539 | 1091 |
| 23 | + | 2449 | >2500 |
| 24 | + | 1923 | 1605 |
| 25 | + | 1420 | 654 |
| 26 | Primär + | 1194 | 193 |
| 27 | Zoster + | 2087 | 1719 |
| 28 | + | 549 | 714 |
| 29 | - | 34 | 207 |
| 30 | - | 76 | 88 |
| 31 | - | 0 | 53 |
| 32 | - | 41 | 89 |
| 33 | - | 8 | 157 |
| 34 | - | 59 | 334 |
| 35 | + | 169 | 150 |
| 36 | + | 1613 | 1187 |
| 37 | - | 3 | 30 |
| 38 | - | 20 | 35 |
| 39 | - | 2 | 56 |
| 40 | - | 0 | 56 |
| 41 | + | 1254 | 1919 |
| 42 | - | 25 | 50 |
| 43 | - | 15 | 43 |
| 44 | - | 58 | 38 |
| 45 | - | 13 | 32 |
| 46 | - | 64 | 61 |
| 47 | - | 68 | 49 |
| 48 | + | 1315 | 751 |
| 49 | + | 1629 | 1423 |
| 50 | + | 469 | 142 |
| 51 | + | 693 | 813 |
| 52 | + | 1449 | 724 |
| 53 | + | 1139 | 1170 |
| 54 | + | 1509 | 537 |
| 55 | + | 457 | 144 |
| 56 | + | 343 | 102 |
| 57 | + | 1455 | 1233 |
| 58 | + | 1030 | 94 |
| 59 | + | 528 | 122 |
| 60 | + | 510 | 122 |
| 61 | + | 300 | 113 |
| 62 | + | 490 | 152 |
| 63 | + | 888 | 308 |
| 64 | + | 1301 | 1087 |
| 65 | + | 704 | 549 |
| 66 | + | 2523 | 802 |
| 67 | + | 447 | 139 |
| 68 | + | 848 | 215 |

**Tabelle 3:**

| **Nr.** | **Status** | **ELISA IgM Referenz** | **ELISA IgM pQEgpII 4 µg/ml** |
|---|---|---|---|
| | | **Konj. 1:50** | **Konj. 1:25** |
| | | **Serum** | **Serum** |
| | | **1:42** | **1:100** |
| 1 | - | 11 | 41 |
| 2 | - | 22 | 54 |
| 3 | - | 16 | 77 |
| 4 | - | 57 | 80 |
| 5 | + | 133 | 70 |
| 6 | - | 0 | 38 |
| 7 | - | 10 | 55 |
| 8 | - | 47 | 51 |
| 9 | - | 7 | 51 |
| 10 | + | 132 | 63 |
| 11 | - | 71 | 66 |
| 12 | - | 0 | 57 |
| 13 | - | 17 | 39 |
| 14 | - | 11 | 53 |
| 15 | - | 23 | 45 |
| 16 | - | 11 | 52 |
| 17 | + | 979 | 270 |
| 18 | - | 9 | 42 |
| 19 | - | 34 | 73 |
| 20 | + | 293 | 232 |
| 21 | + | 126 | 117 |
| 22 | - | 61 | 86 |
| 23 | + | 292 | 49 |
| 24 | + | 581 | 54 |
| 25 | + | 321 | 77 |
| 26 | + | 509 | 155 |
| 27 | + | 298 | 80 |
| 28 | - | 29 | 72 |
| 29 | - | 16 | 55 |
| 30 | - | 8 | 44 |
| 31 | - | 3 | 48 |
| 32 | - | 50 | 48 |
| 33 | - | 9 | 72 |
| 34 | + | 250 | 131 |
| 35 | + | 291 | 68 |
| 36 | - | 26 | 40 |
| 37 | + | 139 | 63 |
| 38 | - | 38 | 35 |
| 39 | - | 26 | 49 |
| 40 | - | 49 | 55 |
| 41 | - | 5 | 37 |
| 42 | - | 27 | 96 |
| 43 | - | 51 | 40 |
| 44 | - | 8 | 43 |
| 45 | - | 21 | 39 |
| 46 | - | 10 | 49 |
| 47 | - | 29 | 38 |
| 48 | - | 30 | 67 |
| 49 | - | 21 | 34 |
| 50 | - | 56 | 82 |
| 51 | - | 5 | 56 |
| 52 | - | 97 | 24 |
| 53 | - | 2 | 28 |
| 54 | - | 31 | 43 |

## Patentansprüche

1. Immunchemisches Verfahren zum Nachweis von Antikörpern gegen Varicella-Zoster-Virus (VZV) in einer Probe mittels eines immunreaktiven Peptids, welches dem Bereich von AS 450 bis 655 des gpll des VZV entspricht.

2. Immunchemisches Verfahren gemäß Anspruch 1, worin das immunreaktive Peptid dem Bereich von AS 505 bis 647 des gpII des VZV entspricht.

3. Immunchemisches Verfahren gemäß Anspruch 2, worin das immunreaktive Peptid dem Bereich von AS 517 bis 597 des gpII des VZV entspricht.

4. Immunchemisches Verfahren gemäß Anspruch 3, worin das immunreaktive Peptid dem Bereich von AS 535 bis 584 des gpII des VZV entspricht.

5. Immunreaktives Peptid, welches dem Bereich von AS 535 bis 584 des gpII des VZV entspricht.

6. Testbesteck zum Nachweis von Antikörpern gegen VZV, enthaltend ein immunreaktives Peptid, welches einem Bereich des gpII des VZV entspricht, ausgewählt aus den folgenden Bereichen: AS 450 bis 655, AS 505 bis 647, AS 517 bis 597 und AS 535 bis 584.

## Claims

1. An immunochemical method of detecting antibodies to varicella zoster virus (VZV) in a sample by means of an immunoreactive peptide which corresponds to the region encompassing AA 450 to 655 of gpII of VZV.

2. The immunochemical method as claimed in claim 1, wherein the immunoreactive peptide corresponds to the region encompassing AA 505 to 647 of gpII of VZV.

3. The immunochemical method as claimed in claim 2, wherein the immunoreactive peptide corresponds to the region encompassing AA 517 to 597 of gpII of VZV.

4. The immunochemical method as claimed in claim 3, wherein the immunoreactive peptide corresponds to the region encompassing AA 535 to 584 of gpII of VZV.

5. An immunoreactive peptide corresponding to the region encompassing AA 535 to 584 of gpII of VZV.

6. A test kit for detecting antibodies to VZV, comprising an immunoreactive peptide corresponding to a region of gpII of VZV selected from the following regions: AA 450 to 655, AA 505 to 647, AA 517 to 597 and AA 535 to 584.

## Revendications

1. Procédé immunochimique de détection d'anticorps à l'encontre du virus varicella-zoster ( VZV ) dans un échantillon au moyen d'un peptide immunoréactif qui correspond à la partie allant de AS 450 à 655 du gpll du VZV.

2. Procédé immunochimique suivant la revendication 1, dans lequel le peptide immunoréactif correspond à la partie allant de AS 505 à 647 du gpll du VZV.

3. Procédé immunochimique suivant la revendication 2, dans lequel le peptide immunoréactif correspond à la partie allant de AS 517 à 597 du gpll du VZV.

4. Procédé immunochimique suivant la revendication 3, dans lequel le peptide immunoréactif correspond à la partie allant de AS 535 à 584 du gpll du VZV.

5. Peptide immunoréactif qui correspond à la partie allant de AS 535 à 584 du gpll du VZV.

6. Trousse de test pour la détection d'anticorps à l'encontre de VZV, comprenant un peptide immunoréactif qui correspond à une partie du gpll du VZV choisie dans les parties suivantes : de AS 450 à 655, de AS 505 à 647, de AS 517 à 597 et de AS 535 à 584.


### SEQUENZPROTOKOLL

### (1) ALLGEMEINE ANGABEN:

(i) ANMELDER:
   (A) NAME: Dade Behring Marburg GmbH
   (B) STRASSE: Emil-von-Behring-Str. 76
   (C) ORT: Marburg
   (E) LAND: Deutschland
   (F) POSTLEITZAHL: 35001
   (G) TELEFON: 06421/39-2332
   (H) TELEFAX: 06421/39-3631
(ii) BEZEICHNUNG DER ERFINDUNG: Immunreaktive Bereiche des Glycoproteins gpII des Varicella-Zoster-Virus (VZV)
(iii) ANZAHL DER SEQUENZEN: 10
(iv) COMPUTER-LESBARE FASSUNG:
   (A) DATENTRÄGER: Floppy disk
   (B) COMPUTER: IBM PC compatible
   (C) BETRIEBSSYSTEM: PC-DOS/MS-DOS
   (D) SOFTWARE: PatentIn Release #1.0, Version #1.30 (EPA)
(v) DATEN DER JETZIGER ANMELDUNG: ANMELDENUMMER: DE 197 57 767.9

### (2) ANGABEN ZU SEQ ID NO: 1:

(i) SEQUENZKENNZEICHEN:
   (A) LÄNGE: 206 Aminosäuren
   (B) ART: Aminosäure
   (C) STRANGFORM:
   (D) TOPOLOGIE: linear
(ii) ART DES MOLEKÜLS: Protein
(iii) HYPOTHETISCH: JA
(vi) URSPRÜNLICHE HERKUNFT:
   (A) ORGANISMUS: Varizella Zoster Virus
   (B) STAMM: Ellen
(viii) POSITION IM GENOM:
   (B) KARTENPOSITION: ORF31
(xi) SEQUENZBESCHREIBUNG: SEQ ID NO: 1:
   Glu Phe Ala Met Leu Gln Phe Thr Tyr Asp His Ile Gln Glu His Val
   1 5 10 15
   Asn Glu Met Leu Ala Arg Ile Ser Ser Ser Trp Cys Gln Leu Gln Asn
   20 25 30
   Arg Glu Arg Ala Leu Trp Ser Gly Leu Phe Pro Ile Asn Pro Ser Ala
   35 40 45
   Leu Ala Ser Thr Ile Leu Asp Gln Arg Val Lys Ala Arg Ile Leu Gly
   50 55 60
   Asp Val Ile Ser Val Ser Asn Cys Pro Glu Leu Gly Ser Asp Thr Arg
   65 70 75 80
   Ile Ile Leu Gln Asn Ser Met Arg Val Ser Gly Ser Thr Thr Arg Cys
   85 90 95
   Tyr Ser Arg Pro Leu Ile Ser Ile Val Ser Leu Asn Gly Ser Gly Thr
   100 105 110
   Val Glu Gly Gln Leu Gly Thr Asp Asn Glu Leu Ile Met Ser Arg Asp
   115 120 125
   Leu Leu Glu Pro Cys Val Ala Asn His Lys Arg Tyr Phe Leu Phe Gly
   130 135 140
   His His Tyr Val Tyr Tyr Glu Asp Tyr Arg Tyr Val Arg Glu Ile Ala
   145 150 155 160
   Val His Asp Val Gly Met Ile Ser Thr Tyr Val Asp Leu Asn Leu Thr
   165 170 175
   Leu Leu Lys Asp Arg Glu Phe Met Pro Leu Gln Val Tyr Thr Arg Asp
   180 185 190
   Glu Leu Arg Asp Thr Gly Leu Leu Asp Tyr Ser Glu Ile Gln
   195 200 205

### (2) ANGABEN ZU SEQ ID NO: 2:

(i) SEQUENZKENNZEICHEN:
   (A) LÄNGE: 618 Basenpaare
   (B) ART: Nucleotid
   (C) STRANGFORM: Einzelstrang
   (D) TOPOLOGIE: linear
(ii) ART DES MOLEKÜLS: Genom-DNA
(iii) HYPOTHETISCH: JA
(vi) URSPRÜNLICHE HERKUNFT:
   (A) ORGANISMUS: Varizella Zoster Virus
   (B) STAMM: ellen
(viii) POSITION IM GENOM:
   (B) KARTENPOSITION: ORF31
(xi) SEQUENZBESCHREIBUNG: SEQ ID NO: 2:
   GAATTTGCTA TGCTCCAGTT TACATATGAC CACATTCAAG AGCATGTTAA TGAAATGTTG     60
   GCACGTATCT CCTCGTCGTG GTGCCAGCTA CAAAATCGCG AACGCGCCCT TTGGAGCGGA    120
   CTATTTCCAA TTAACCCAAG TGCTTTAGCG AGCACCATTT TGGATCAACG TGTTAAAGCT    180
   CGTATTCTCG GCGACGTTAT CTTCGTTTCT AATTGTCCAG AACTGGGATC AGATACACGC    240
   ATTATACTTC AAAACTCTAT GAGGGTATCT GGTAGTACTA CGCGTTGTTA TAGCCGTCCT    300
   TTAATTTCAA TAGTTAGTTT AAATGGGTCC GGGACGGTGG AGGGCCAGCT TGGAACAGAT    360
   AACGAGTTAA TTATGTCCAG AGATCTGTTA GAACCATGCG TGGCTAATCA CAAGCGATAT    420
   TTTCTATTTG GGCATCACTA CGTATATTAT GAGGATTATC GTTACGTCCG TGAAATCGCA    480
   GTCCATGATG TGGGAATGAT TAGCACTTAC GTAGATTTAA ACTTAACACT TCTTAAAGAT    540
   AGAGAGTTTA TGCCGCTGCA AGTATATACA AGAGACGAGC TGCGGGATAC AGGATTACTA    600
   GACTACAGTG AAATTCAA                                                  618

### (2) ANGABEN ZU SEQ ID NO: 3:

(i) SEQUENZKENNZEICHEN:
   (A) LÄNGE: 31 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Synthetische DNA"
(iii) HYPOTHETISCH: NEIN
(vi) URSPRÜNLICHE HERKUNFT:
   (A) ORGANISMUS: Varizella Zoster Virus
   (B) STAMM: Ellen
(viii) POSITION IM GENOM:
   (B) KARTENPOSITION: ORF31
(xi) SEQUENZBESCHREIBUNG: SEQ ID NO: 3:
   GGAATTCCTT CTATGTTTGT TACGGCGGTT G 31

### (2) ANGABEN ZU SEQ ID NO: 4:

(i) SEQUENZKENNZEICHEN:
   (A) LÄNGE: 34 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Synthetische DNA"
(iii) HYPOTHETISCH: NEIN
(vi) URSPRÜNLICHE HERKUNFT:
   (A) ORGANISMUS: Varizella Zoster virus
(viii) POSITION IM GENOM:
   (B) KARTENPOSITION: ORF31
(xi) SEQUENZBESCHREIBUNG: SEQ ID NO: 4:
   GCTCTAGAGC ATTTACACCC CCGTTACATT CTCG 34

### (2) ANGABEN ZU SEQ ID NO: 5:

(i) SEQUENZKENNZEICHEN:
   (A) LÄNGE: 36 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Synthetische DNA"
(iii) HYPOTHETISCH: NEIN
(vi) URSPRÜNLICHE HERKUNFT:
   (A) ORGANISMUS: Varizella Zoster Virus
(viii) POSITION IM GENOM:
   (B) KARTENPOSITION: ORF31
(xi) SEQUENZBESCHREIBUNG: SEQ ID NO: 5:
   CGGGATCCCG TGTTAAAGCT CGTATTCTCG GCGACG 36

### (2) ANGABEN ZU SEQ ID NO: 6:

(i) SEQUENZKENNZEICHEN:
   (A) LÄNGE: 36 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Synthetische DNA"
(iii) HYPOTHETISCH: NEIN
(vi) URSPRÜNLICHE HERKUNFT:
   (A) ORGANISMUS: Varizella Zoster Virus
   (B) STAMM: Ellen
(viii) POSITION IM GENOM:
   (B) KARTENPOSITION: ORF3-1
(xi) SEQUENZBESCHREIBUNG: SEQ ID NO: 6:
   CCCAAGCTTT AATCCTGTAT CCCGCAGCTC GTCTCT 36

### (2) ANGABEN ZU SEQ ID NO: 7:

(i) SEQUENZKENNZEICHEN:
   (A) LÄNGE: 36 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Synthetische DNA"
(iii) HYPOTHETISCH: NEIN
(vi) URSPRÜNLICHE HERKUNFT:
   (A) ORGANISMUS: Varizella Zoster Virus
   (B) STAMM: Ellen
(viii) POSITION IM GENOM:
   (B) KARTENPOSITION: ORF31
(xi) SEQUENZBESCHREIBUNG: SEQ ID NO: 7:
   CGGGATCCGT TTCTAATTGT CCAGAACTGG GATCAG 36

### (2) ANGABEN ZU SEQ ID NO: 8:

(i) SEQUENZKENNZEICHEN:
   (A) LÄNGE: 35 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Synthetische DNA"
(iii) HYPOTHETISCH: NEIN
(vi) URSPRÜNLICHE HERKUNFT:
   (A) ORGANISMUS: Varizella Zoster Virus
   (B) STAMM: Ellen
(viii) POSITION IM GENOM:
   (B) KARTENPOSITION: ORF31
(xi) SEQUENZBESCHREIBUNG: SEQ ID NO: 8:
   CCCAAGCTTA TACGTAGTGA TGCCCAAATA GAAAA 35

### (2) ANGABEN ZU SEQ ID NO: 9:

(i) SEQUENZKENNZEICHEN:
   (A) LÄNGE: 39 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Synthetische DNA"
(iii) HYPOTHETISCH: NEIN
(vi) URSPRÜNLICHE HERKUNFT:
   (A) ORGANISMUS: Varizella Zoster virus
   (B) STAMM: Ellen
(viii) POSITION IM GENOM:
   (B) KARTENPOSITION: Orf31
(xi) SEQUENZBESCHREIBUNG: SEQ ID NO: 9:
   CGGGATCCTC TATGAGGGTA TCTGGTAGTA CTACGCGTT 39

### (2) ANGABEN ZU SEQ ID NO: 10:

(i) SEQUENZKENNZEICHEN:
   (A) LÄNGE: 33 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Synthetische DNA"
(iii) HYPOTHETISCH: NEIN
(vi) URSPRÜNLICHE HERKUNFT:
   (A) ORGANISMUS: varizella Zoster Virus
   (B) STAMM: Ellen
(viii) POSITION IM GENOM:
   (B) KARTENPOSITION: ORF31
(xi) SEQUENZBESCHREIBUNG: SEQ ID NO: 10:
   CCCAAGCTTA GCCACGCATG GTTCTAACAG ATC 33